# EUROPEAN PATENT APPLICATION

(11) **EP 4 084 015 A1**
(43) Date of publication of application: **02.11.2022**
(21) Application number: 20906573.9
(22) Date of filing: 11.12.2020
(51) Int. Cl.: G16H 80/00

(54) **CONTROL METHOD, PROGRAM, AND CONTROL DEVICE**

(30) Priority: 27.12.2019 US 201962954225 P
(71) Applicant: Panasonic Intellectual Property Corporation of America, Torrance, CA 90503 (US)
(72) Inventor: MITANI, Ayaka, Osaka-shi Osaka 540-6207 (JP); UNAGAMI, Yuji, Osaka-shi Osaka 540-6207 (JP); FUCHIKAMI, Tetsuji, Osaka-shi Osaka 540-6207 (JP)
(74) Representative: Novagraaf International SA
(86) International application number: PCT/JP2020/046287
(87) International publication number: WO 2021/131794

(57) **Abstract**

A control method according to the present disclosure is to be executed by a system including a distributed ledger storing a smart contract obtained by programming, in an executable manner, a determination on a medical treatment made as a result of a discussion between a patient and a physician. The control method includes: obtaining condition transaction data including biological information on the patient obtained by a sensor (S205); obtaining opinion transaction data including information on a result of a diagnosis by the physician based on the biological information (S210); executing the smart contract based on the biological information and the result of the diagnosis included in the condition transaction data and the opinion transaction data obtained, respectively; and outputting medical suggestion information indicating a medical treatment to be given to the patient, and obtained by executing the smart contract (S211 and S212).

## Description

### [Technical Field]

The present disclosure relates to a control method, a program, and a control device employing the blockchain technology.

### [Background Art]

For example, Patent Literature (PTL) 1 suggests a medical determination support system that evaluates known medical facts accumulated in a server and selects the most effective medical suggestion based on the evaluation.

Here, the medical facts include, for example, a medical diagnosis, medical observations, a drug treatment, a medical history, secondary suggestions, an evaluation on test results, a medical adequacy, a medical conclusion, a medical treatment, medical instructions, a medical report, medical questions, a symptom, a syndrome, a text block, or nutritional recommendations. The medical facts may also include, for example, exercise suggestions, healthcare suggestions, rehabilitation suggestions, genetic aspects, histology observations, findings in physiological and pathophysiological processes, quality indexes, treatment recommendations, therapeutic recommendations, process suggestions, a medical survey suggestion, a questionnaire to the patient, or a questionnaire to a professional. Note that the medical facts may include any combination of these examples.

For example, PTL 2 suggests a method of sharing and managing medical information including the decision indicated in advance by a patient via the Internet in view of the reliability and privacy.

In the techniques suggested in PTL 1 and PTL 2 described above, however, a medical treatment can be determined only when a patient himself/herself can indicate his/her intention. That is, no medical treatment can be determined in view of the decision of the patient, when the patient is unable to indicate his/her intention.

On the other hand, there is a method of determining a medical treatment in view of the decision of a patient, using a living will or a POLST form reflecting the decision indicated in advance by the patient or others, even when the patient himself/herself is unable to indicate his/her intention (see, e.g., Non Patent Literature (NPL 1)). Here, the POLST stands for "Physician Orders for Life-Sustaining Treatment". The POLST are the physician orders for medical treatments including a resuscitation treatment in the end of life of a patient, and includes a Do Not Attempt Resuscitate (DNAR) order. Based on the POLST, a physician determines a medical treatment to be given to a patient who has a "life threatening illness" including as to whether a cardiopulmonary resuscitation treatment is to be given. Assume that there is a special order, such as the DNAR order, for performing no cardiopulmonary resuscitation after the stop of the breathing or heartbeat of a patient with terminal cancer. In this case, the physician can omit the cardiopulmonary resuscitation in accordance with the DNAR order.

### [Citation List]

### [Patent Literatures]

[PTL 1] Japanese Unexamined Patent Application Publication (Translation of PCT Application) No. 2016-516231
[PTL 2] Japanese Unexamined Patent Application Publication (Translation of PCT Application) No. 2015-507282

### [Non Patent Literature]

[NPL 1] "Physician Orders for Life-Sustaining Treatment (POLST) and Do Not Attempt Resuscitation (DNAR) Order" https://www.jsicm.org/pdf/DNAR20161216_kangae_02.pdf (2020/03/13)

### [Summary of Invention]

### [Technical Problems]

Even with the use of the POLST disclosed in NPL 1, however, whether a medical treatment has been determined and given in view of the decision of a patient when the patient himself/herself was unable to indicate his/her intention cannot be verified. That is, when a patient is unable to indicate his/her intention actually, whether an actual medical treatment was given in view of the decision of the patient reflected in advance in the POLST, for example, cannot be verified.

Under such circumstances, if a medical treatment ignoring the decision by the patient was given, the adequacy of the medical treatment cannot be verified and where the responsibility for the treatment lies is unclear.

The present disclosure was made under the circumstances. It is an objective of the present disclosure to provide a control method, a program, and a control device capable of verifying whether a medical treatment has been determined and given in view of the decision indicated in advance by a patient or others.

### [Solutions to Problems]

In order to achieve the objective, a control method according to an aspect of the present disclosure is to be executed by a system including a distributed ledger storing a smart contract obtained by programming, in an executable manner, a determination on a medical treatment made as a result of a discussion between a patient and a physician. The control method includes: obtaining first transaction data including biological information on the patient obtained by a sensor; obtaining second transaction data including information on a result of a diagnosis by the physician based on the biological information; executing the smart contract based on the biological information and the result of the diagnosis included in the first transaction data and the second transaction data obtained, respectively; and outputting medical suggestion information indicating a medical treatment to be given to the patient, and obtained by executing the smart contract.

This general and specific aspect may be implemented using a system, an integrated circuit, a computer program, or a computer-readable recording medium such as a CD-ROM, or any combination of systems, methods, integrated circuits, computer programs, or recording media.

### [Advantageous Effects of Invention]

It is an objective of the present disclosure to provide a control method, a program, and a control device capable of verifying whether a medical treatment has been determined and given in view of the decision indicated in advance by a patient or others.

### [Brief Description of Drawings]

[FIG. 1] FIG. 1 shows an overall configuration of a medical treatment management system according to an embodiment.
[FIG. 2] FIG. 2 is a block diagram showing an example configuration of a patient terminal according to the embodiment.
[FIG. 3] FIG. 3 illustrates a data structure of a blockchain.
[FIG. 4] FIG. 4 is a block diagram showing an example configuration of a physician terminal according to the embodiment.
[FIG. 5] FIG. 5 shows an address configuration in a blockchain network held in a distributed ledger according to the embodiment.
[FIG. 6] FIG. 6 conceptionally shows a data structure of third transaction data according to the embodiment.
[FIG. 7] FIG. 7 conceptionally shows a data structure of condition transaction data according to the embodiment.
[FIG. 8] FIG. 8 conceptionally shows a data structure of opinion transaction data according to the embodiment.
[FIG. 9] FIG. 9 conceptionally shows an algorithm of a medical determination support smart contract according to the embodiment.
[FIG. 10] FIG. 10 conceptionally shows an algorithm of a medical determination support smart contract according to Example 1.
[FIG. 11] FIG. 11 conceptionally shows an algorithm of a medical determination support smart contract according to Example 2.
[FIG. 12] FIG. 12 is a block diagram showing an example configuration of a server device according to the embodiment.
[FIG. 13A] FIG. 13A shows an outline of an operation until storing a decision indicated by a patient in the distributed ledger of the medical treatment management system according to the embodiment.
[FIG. 13B] FIG. 13B shows an outline of an operation until recording whether a medical treatment has been determined and given in accordance with the decision by the patient stored in the distributed ledger of the medical treatment management system according to the embodiment.
[FIG. 14] FIG. 14 shows a sequence of generating and activating the medical determination support smart contract using the medical treatment management system according to the embodiment.
[FIG. 15] FIG. 15 shows a sequence until executing the medical determination support smart contract and recording a result of the medical treatment according to the embodiment. [Description of Embodiment]

A control method according to an aspect of the present disclosure is to be executed by a system including a distributed ledger storing a smart contract obtained by programming, in an executable manner, a determination on a medical treatment made as a result of a discussion between a patient and a physician. The control method includes: obtaining first transaction data including biological information on the patient obtained by a sensor; obtaining second transaction data including information on a result of a diagnosis by the physician based on the biological information; executing the smart contract based on the biological information and the result of the diagnosis included in the first transaction data and the second transaction data obtained, respectively; and outputting medical suggestion information indicating a medical treatment to be given to the patient, and obtained by executing the smart contract.

In this manner, a smart contract suggests for a medical treatment in case where the patient himself/herself is unable to indicate his/her intention. The smart contract uniquely determines a suggestion for a medical treatment based on the medical condition or other factors of the patient in view of the decision indicated in advance by the patient. Accordingly, the actually given medical treatment can be compared later to the medical treatment suggested by the smart contract. As a result, whether the medical treatment has been determined and given in view of the decision indicated in advance by the patient can be verified.

For example, the control method may further include, before obtaining the first transaction data and the second transaction data: obtaining third transaction data including the smart contract generated based on the determination on the medical treatment made as the result of the discussion between the patient and the physician; and storing, in the distributed ledger, a block including the third transaction data.

Accordingly, the smart contract is generated in view of the decision indicated in advance by the patient, and stored in the distributed ledger.

Here, for example, the third transaction data may include a signature for verifying an authenticity of the third transaction data.

For example, the smart contract may be a program including a description including a conditional expression for executing the determination on the medical treatment, using the first transaction data and the second transaction data as input values.

For example, each of the first transaction data and the second transaction data may include a contract address indicating an address of the smart contract.

For example, the control method may further include: obtaining fourth transaction data including medical treatment result information which is information on a medical treatment given to the patient by the physician; and storing, in the distributed ledger, the medical treatment result information in association with the medical suggestion information.

In this method, the actually given medical treatment is stored in the distributed ledger. Accordingly, the actually given medical treatment can be compared to the medical treatment suggested by the smart contract more reliably. As a result, whether a medical treatment has been determined and given in view of the decision indicated in advance by the patient can be verified.

Here, for example, the biological information may be information indicating a cardiopulmonary arrest. The result of the diagnosis may indicate that there is no external injury. The medical suggestion information may be information indicating that the medical treatment to be given to the patient is cardiopulmonary resuscitation.

Alternatively, the distributed ledger may be constructed on a blockchain platform.

A control device according to an aspect of the present disclosure includes: a processor; a memory; and a distributed ledger. The distributed ledger stores a smart contract obtained by programming, in an executable manner, a determination on a medical treatment made as a result of a discussion between a patient and a physician. The processor obtains first transaction data includes biological information on the patient obtained by a sensor. The processor obtains second transaction data including information on a result of a diagnosis by the physician based on the biological information. The processor executes the smart contract based on the biological information and the result of the diagnosis included in the first transaction data and the second transaction data obtained, respectively, using the memory. The processor outputs medical suggestion information indicating a medical treatment to be given to the patient, and obtained by executing the smart contract.

An embodiment will be described with reference to the drawings. Note that the embodiment described below is a mere preferred example of the present disclosure. The numerical values, shapes, materials, constituent elements, the arrangement and connection of the constituent elements, steps, step orders etc. shown in the following embodiment are thus mere examples, and are not intended to limit the scope of the present disclosure. The present disclosure is defined based on claim recitations. Among the constituent elements in the following embodiment, those not recited in any of the independent claims defining the broadest concept of the present disclosure are not necessarily essential to achieve the objective of the present disclosure but will be described as constituent elements forming more preferred embodiments.

### [Embodiment]

First, a system configuration according to the present disclosure will be described.

### [1. System Configuration]

The medical treatment management system according to the present disclosure records the decision indicated by a patient in a highly reliable and transparent distributed ledger constructed on a blockchain platform, causes a smart contract on the blockchain to autonomously determine a medical treatment in view of the decision indicated in advance by the patient, and outputs the medical treatment as medical suggestion information. The medical treatment management system according to the present disclosure records the determined medical treatment and the medical treatment given by the physician in association with each other in the distributed ledger. Accordingly, whether the physician has determined and given the medical treatment in view of the decision indicated in advance by the patient or others can be verified highly reliably.

Now, the medical treatment management system according to the embodiment will be described with reference to the drawings.

### [1.1 Overall Configuration of Medical Treatment Management System 1]

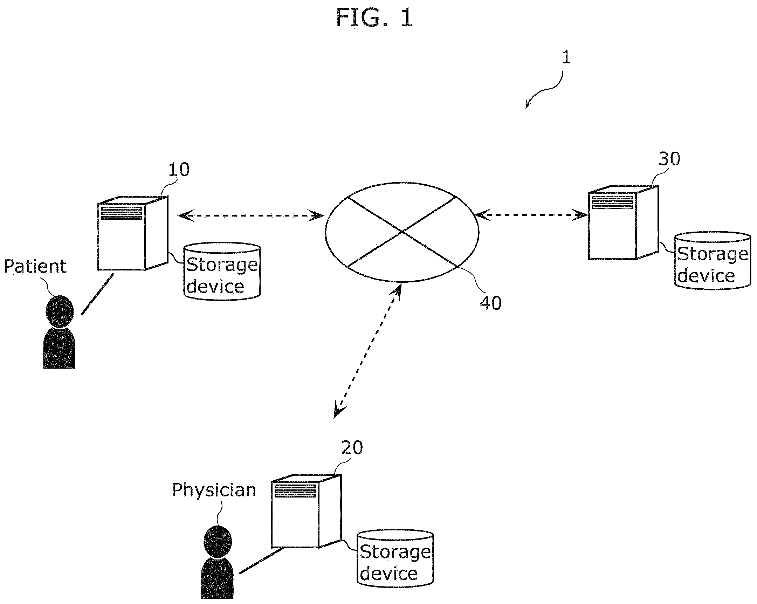

FIG. 1 shows an overall configuration of medical treatment management system 1 according to this embodiment.

As shown in FIG. 1, medical treatment management system 1 includes one or more patient terminals 10, one or more physician terminals 20, and one or more server devices 30. These are connected by communication network 40.

One or more patient terminals 10, each of one or more physician terminals 20, and one or more server devices 30 is connected to a storage device including a distributed ledger electronically recording transaction data and blocks of a blockchain. Each of one or more patient terminals 10, one or more physician terminals 20, and one or more server devices 30 may be connected via communication network 40 to the storage device or include the storage device as a storage.

In this manner, one or more patient terminals 10, one or more physician terminals 20, and one or more server devices 30 constitute a blockchain network.

### [1.2 Configuration of Patient Terminal 10]

Patient terminal 10 is, for example, a terminal of a personal computer or other devices used by a patient but not limited thereto. Patient terminal 10 is not necessary a terminal but may be a server, as long as including the distributed ledger, capable of constituting the blockchain network, and being used by the patient or directly communicative with the patient.

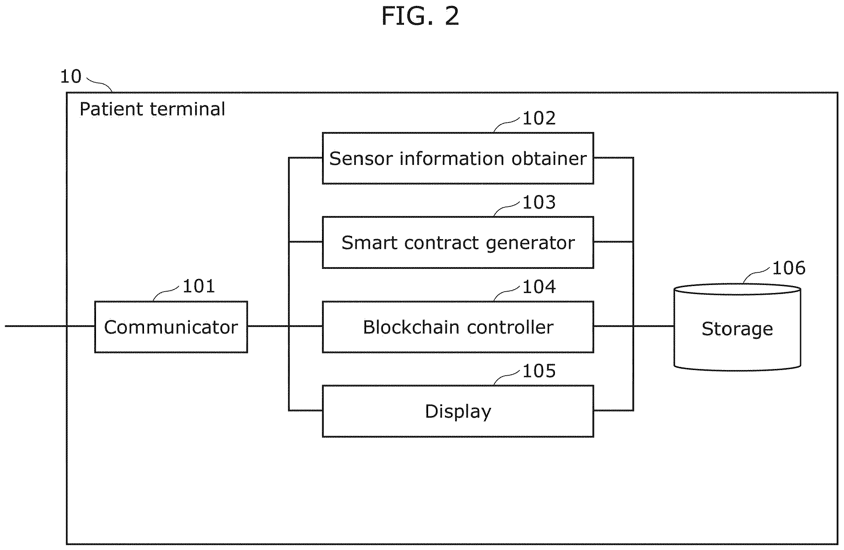

FIG. 2 is a block diagram showing an example configuration of patient terminal 10 according to this embodiment.

As shown in FIG. 2, patient terminal 10 includes communicator 101, sensor information obtainer 102, smart contract generator 103, blockchain controller 104, display 105, and storage 106.

### <Communicator 101>

Communicator 101 communicates via communication network 40 with physician terminal 20 and server device 30. Communicator 101 also communicates with an input device, such as a keyboard and a microphone, used by a patient.

For example, communicator 101 obtains the text and/or audio data input under an operation by the patient, for example, and obtains the data input by the patient interactive with display 105.

<Sensor Information Obtainer 102>

Sensor information obtainer 102 obtains sensor information that is the information from a sensor group connected via communicator 101 to patient terminal 10. Here, the sensor information is used to indicate the medical condition of the patient, and indicates the medical facts. Sensor information may be the biological information on the patient obtained by the sensor group. The biological information is the vital data on the patient including the heart rate, the blood glucose level, the cognitive function evaluation, and the respiratory rate of the patient.

Sensor information obtainer 102 transmits the obtained sensor information to smart contract generator 103, blockchain controller 104, display 105, or storage 106 depending on the content.

### <Smart Contract Generator 103>

Smart contract generator 103 generates a medical determination support smart contract based on a determination on a medical treatment made as a result of a discussion between the patient and the physician. Here, the smart contract is a program that is executable on a blockchain and provides a unique output in response to a unique input value. The medical determination support smart contract is obtained by programming, in an executable manner, the determination on the medical treatment made as a result of a discussion between the patient and the physician. When the medical determination support smart contract is executed, medical suggestion information on a medical treatment to be given to the patient is output.

In this embodiment, smart contract generator 103 programs an algorithm of the medical determination support smart contract using data such as a medical suggestion subset obtained via communicator 101 to generate the medical determination support smart contract. Smart contract generator 103 then transmits the generated medical determination support smart contract to blockchain controller 104. Note that the medical suggestion subset is data indicating a combination of suggestable medical treatments.

Here, an example method generating a medical determination support smart contract will be described.

First, smart contract generator 103 obtains data such as a medical suggestion subset via communicator 101 from server device 30. Next, smart contract generator 103 obtains data indicating the content of the medical determination support smart contract input by the patient interactive with display 105 while discussing with the patient. Based on the medical suggestion subset, smart contract generator 103 generates a medical determination support smart contract. More specifically, smart contract generator 103 obtains the following conditional expression, variables, and constants using, as input values, the sensor information obtained from patient terminal 10 and the medical facts obtained from physician terminal 20. The conditional expression is for making determination on the input values. The constants form the conditional expression. The variables and constants correspond to the inputs and the output results of the conditional expression. Using the obtained conditional expression, variables, and constants, smart contract generator 103 then updates the program to be executable on the blockchain and to provide a unique output in response to a unique input value. Accordingly, the medical determination support smart contract is generated.

In this embodiment, smart contract generator 103 generates, as a binarized program, a conditional expression described by Solidity, which is a scripting language executable on an Ethereum-based blockchain, and how to access the obtained constants and variables. Smart contract generator 103 then transmits the generated, binarized program as the medical determination support smart contract to blockchain controller 104.

### <Blockchain Controller 104>

Blockchain controller 104 controls the establishment of the blockchain network, the generation of transaction data, the synchronization of the distributed ledger, and the execution of a consensus algorithm. Blockchain controller 104 also controls the storage of the respective control results in storage 106.

In this embodiment, blockchain controller 104 generates the transaction data including sensor information received by sensor information obtainer 102. For example, the sensor information includes the biological information on the patient and is obtained by sensors. For example, blockchain controller 104 generates the transaction data including the medical determination support smart contract generated by smart contract generator 103. Note that the transaction data including the sensor information obtained by a sensor and including the biological information on the patient will be hereinafter referred to as "transaction data indicating the medical condition of the patient" or "condition transaction data". The transaction data including the medical determination support smart contract will also be referred to as "third transaction data". When generating the condition transaction data, blockchain controller 104 includes, as a destination address, a contract address indicating the address of the medical determination support smart contract. Details thereof will be described later.

Here, a data structure of the blockchain will be described.

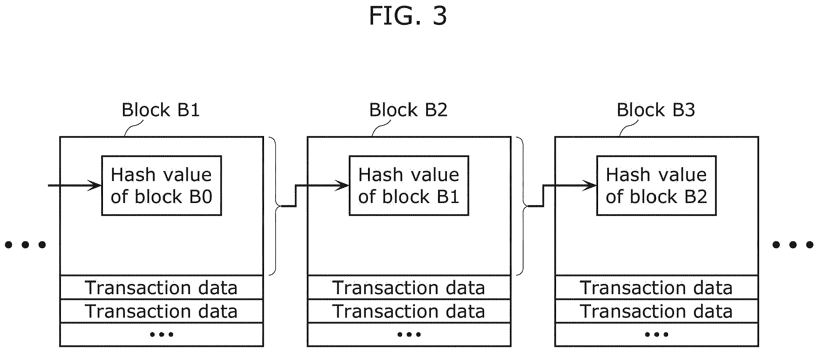

FIG. 3 illustrates the data structure of the blockchain.

The blockchain is obtained by connecting blocks, which are recording units, in a chain. Each block includes a plurality of transaction data and the hash value of a block immediately before the block. Specifically, block B2 includes the hash value of block B1 immediately before block B2. The hash value calculated from the plurality of transaction data included in block B2 and the hash value of block B1 are included as the hash value of block B2 in block B3. In this manner, the blocks are connected in a chain, while including the contents of the previous blocks as hash values, to effectively reduce the falsification of the connected transaction data.

If past transaction data is changed, the hash value of a block is different from that before the change. In order to make the falsified block look like the correct one, there is a need to recreate all the subsequent blocks. This work is practically extremely difficult.

Now, an example will be described where Ethereum assumed to mount a smart contract is selected as the blockchain platform.

Blockchain controller 104 establishes Peer-to-Peer communications under a prescribed blockchain protocol to control the establishment of the blockchain network.

Blockchain controller 104 receives binary data on the medical determination support smart contract generated by smart contract generator 103, and generates transaction data including the binary data as the content data. Blockchain controller 104 also receives the sensor information generated by smart contract generator 103, and generates the transaction data including the sensor information. Although details will be described later, when generating the transaction data, blockchain controller 104 includes, as a destination address, the blockchain address associated with patient terminal 10. Blockchain controller 104 then stores (records) the generated transaction data in the distributed ledger of patient terminal 10.

In this manner, blockchain controller 104 controls the generation of the transaction data. The generated transaction data is received and transmitted between addresses in the blockchain network. Note that the generated transaction data further includes a signature unique to blockchain controller 104 or patient terminal 10.

Blockchain controller 104 executes the consensus algorithm for making the agreement on the validity of the generated transaction data. After reaching the agreement on the validity, blockchain controller 104 records the block including the transaction data in the distributed ledger. In this embodiment, blockchain controller 104 executes the consensus algorithm to reliably take the consensus algorithm into the block and record the consensus algorithm in the distributed ledger.

In Ethereum or other platforms, the smart contract is a code to be deployed on a blockchain. Thus, the transaction data including the binary data on the smart contract is transmitted to the null address to deploy the smart contract. Note that "to deploy the smart contract" is to deploy the program of the smart contract which is the actual form of the smart contract on the blockchain network. On the other hand, the transaction data including information for executing the smart contract or information to be recorded as a history is broadcasted, subjected to the consensus algorithm, and stored in the distributed ledger.

In this embodiment, blockchain controller 104 broadcasts newly generated transaction data to physician terminal 20 and server device 30 which are the other nodes constituting the blockchain network. Blockchain controller 104 then stores the newly generated transaction data in the distributed ledger in synchronization with all the nodes constituting the blockchain network. In addition, blockchain controller 104 executes the consensus algorithm among all the nodes constituting the blockchain network to determine whether the newly generated transaction data is to be taken into the block and fixed.

In this manner, blockchain controller 104 stores a block including third transaction data in the distributed ledger. The third transaction data includes a signature for verifying the authenticity of the third transaction data.

### <Display 105>

Display 105 obtains the text and/or audio data input under an operation of the patient, for example, and interactively displays the data input by the patient. In addition, display 105 may function as an interface including an input device (not shown).

Display 105 also displays questions for making a decision on a medical treatment to be indicated in advance by the patient for the case where the patient is unable to indicate his/her intention, while discussing with the physician, using data such as a medical suggestion subset obtained via communicator 101.

Display 105 also displays a medical treatment determined as a result of a discussion between the patient and the physician. Display 105 displays the content of the medical determination support smart contract generated by smart contract generator 103 understandable to the user such as the patient. Note that display 105 performs not only visual display but also auditorially or tactilely provides the user with the content to be displayed.

### <Storage 106>

Storage 106 stores the sensor information obtained by sensor information obtainer 102, the medical determination support smart contract generated by smart contract generator 103, the text and/or audio data input under an operation of the patient, for example, and the data input by the patient.

Storage 106 may be a storage device shown in FIG. 1 including a distributed ledger. In this case, defined in storage 106 may be a distributed ledger area which includes the distributed ledger.

### [1.3 Configuration of Physician Terminal 20]

Physician terminal 20 is, for example, a terminal of a PC used by a physician, but not limited thereto. Physician terminal 20 is not necessary a terminal but may be a server, as long as including the distributed ledger, capable of constituting the blockchain network, and being used by the physician or directly communicative with the physician.

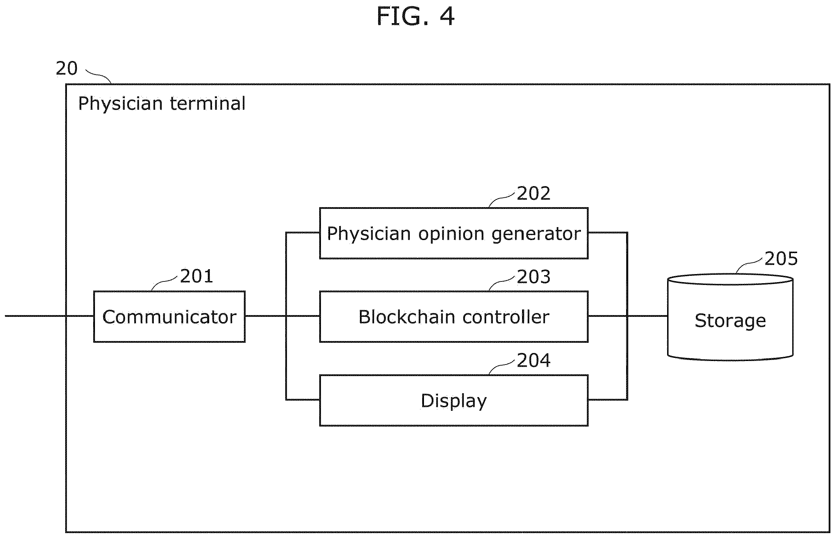

FIG. 4 is a block diagram showing an example configuration of physician terminal 20 according to this embodiment.

As shown in FIG. 4, physician terminal 20 includes communicator 201, physician opinion generator 202, blockchain controller 203, display 204, and storage 205.

### <Communicator 201>

Communicator 201 communicates via communication network 40 with patient terminal 10 and server device 30. Communicator 201 also communicates with an input device, such as a keyboard and a microphone, used by the physician.

For example, communicator 201 obtains the text and/or audio data input under an operation of the physician, for example, and obtains the data input by the physician interactive with display 204.

### <Physician opinion Generator 202>

Physician opinion generator 202 generates, as an opinion of the physician, a result of a diagnosis by the physician based on the data input under an operation of the physician, for example. The result of the diagnosis by the physician indicates the medical facts described above.

In this embodiment, physician opinion generator 202 generates information on a result of a diagnosis by the physician based on the biological information on the patient obtained by sensors. More specifically, physician opinion generator 202 generates the result of the diagnosis by the physician based on the biological information obtained from patient terminal 10. Physician opinion generator 202 converts the generated result of the diagnosis by the physician to a format described with numerical values or an equality sign according to the medical determination support smart contract. Physician opinion generator 202 transmits the converted result of the diagnosis by the physician, as the information on the result of the diagnosis by the physician, to blockchain controller 203.

Physician opinion generator 202 also generates, as an opinion of the physician, medical treatment result information. The medical treatment result information is information on a medical treatment given to the patient by the physician based on the data input under an operation of the physician, for example.

### <Blockchain Controller 203>

Blockchain controller 203 controls the establishment of the blockchain network, the generation of transaction data, the synchronization of the distributed ledger, and the execution of the consensus algorithm. Blockchain controller 203 also controls the storage of the respective control results in storage 205.

In this embodiment, blockchain controller 203 generates the transaction data including information on the result of the diagnosis by the physician generated by physician opinion generator 202. For example, blockchain controller 203 generates transaction data including the medical treatment result information generated by smart contract generator 103. Note that the transaction data including the information on the result of the diagnosis by the physician will be hereinafter referred to as "transaction data indicating the opinion of the physician" or "opinion transaction data". The transaction data including the medical treatment result information will be referred to as "medical treatment result transaction data". When generating the opinion transaction data, blockchain controller 203 includes, as a destination address, a contract address indicating the address of the medical determination support smart contract. Details thereof will be described later.

Note that blockchain controller 203 performs the other controls like blockchain controller 104 of patient terminal 10. The detailed description thereof will thus be omitted here.

### <Display 204>

Display 204 obtains the text and/or audio data input under an operation of the physician, for example, and interactively displays the data input by the physician. In addition, display 204 may function as an interface including an input device (not shown).

Display 204 may also display questions displayed on patient terminal 10 in synchronization with patient terminal 10, using data such as a medical suggestion subset obtained via communicator 101. The questions displayed by patient terminal 10 are for making a decision on a medical treatment to be indicated in advance by the patient for the case where the patient is unable to indicate his/her intention as described above.

### <Storage 205>

Storage 205 stores the opinion of the physician generated by physician opinion generator 202, the text and/or audio data input under an operation of the physician, for example, and the data input by the patient.

Storage 205 may be a storage device shown in FIG. 1 including a distributed ledger. In this case, defined in storage 205 may be a distributed ledger area which includes the distributed ledger.

### [1.4 Address Configuration in Blockchain Network]

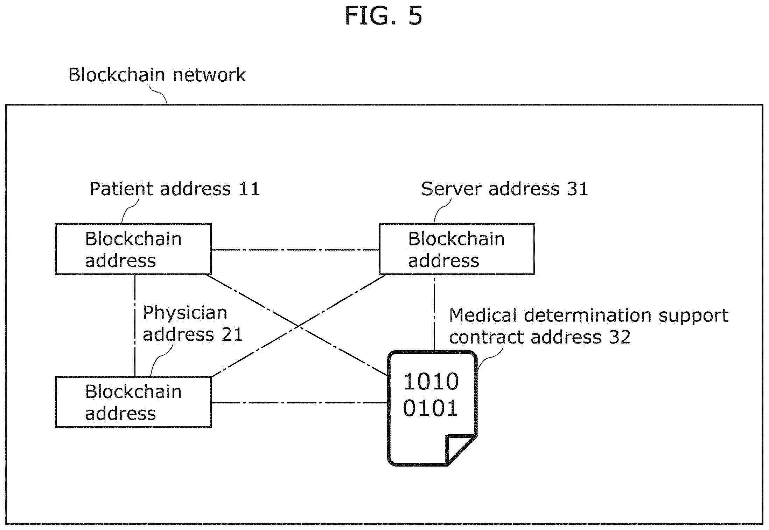

FIG. 5 shows an address configuration in a blockchain network held in a distributed ledger according to this embodiment.

A blockchain address is information given to each of the nodes constituting the blockchain network and uniquely identifies the node. Patient address 11 shown in FIG. 5 is the blockchain address assigned to patient terminal 10. Similarly, physician address 21 shown in FIG. 5 is the blockchain address assigned to physician terminal 20, and server address 31 is the blockchain address assigned to server device 30.

On the other hand, medical determination support contract address 32 shown in FIG. 5 is the unique address assigned to the medical determination support smart contract deployed on the blockchain network. By referring to the unique address, the medical determination support smart contract can be executed.

Note that the address configuration shown in FIG. 5 is as follows. Being held in the distributed ledger, patient address 11, physician address 21, server address 31, and medical determination support contract address 32 are available when generating the transaction data.

### [1.5 Data Structure of Transaction Data]

In this embodiment, the transaction data includes the destination address, the source address, and the content data. As long as including these information, the transaction data may be in any form.

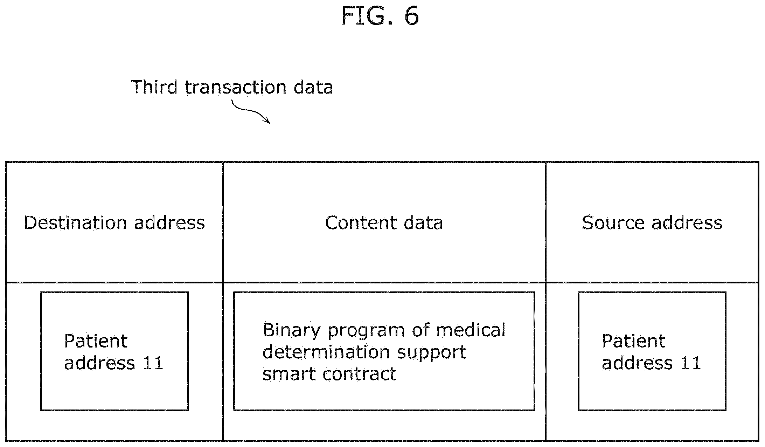

FIG. 6 conceptionally shows a data structure of third transaction data according to this embodiment. The third transaction data includes a medical determination support smart contract, and generated by blockchain controller 104 of patient terminal 10. As described above, the medical determination support smart contract is a program with a description including a conditional expression. The conditional expression is for executing a determination on a medical treatment, using the condition transaction data and the opinion transaction data as input values.

As shown in FIG. 6, the third transaction data includes a destination address, content data, and source address. Each of destination and source addresses includes patient address 11. This is because, in Ethereum or other protocols, there is a need to transmit the transaction data to the null address to deploy the smart contract. The content data includes a binary program of a medical determination support smart contract, that is, a medical determination support smart contract generated as a binarized program. Note that the content data may include a non-binary program as long as being a programmed medical determination support smart contract.

Patient terminal 10 generates third transaction data with such a data structure, and transmits the generated third transaction data to the terminal itself to deploy the medical determination support smart contract in the blockchain network. Note that the content of the third transaction data further includes a signature (not shown) for verifying the authenticity the first transaction data.

Now, data structures of the transaction data serving as inputs to the medical determination support smart contract will be described. The transaction data are, namely, the transaction data indicating the medical condition of the patient and the transaction data indicating the opinion of the physician. The "transaction data indicating the medical condition of the patient" and the "transaction data indicating the opinion of the physician" will be described below while being referred to as "condition transaction data" and "opinion transaction data", respectively.

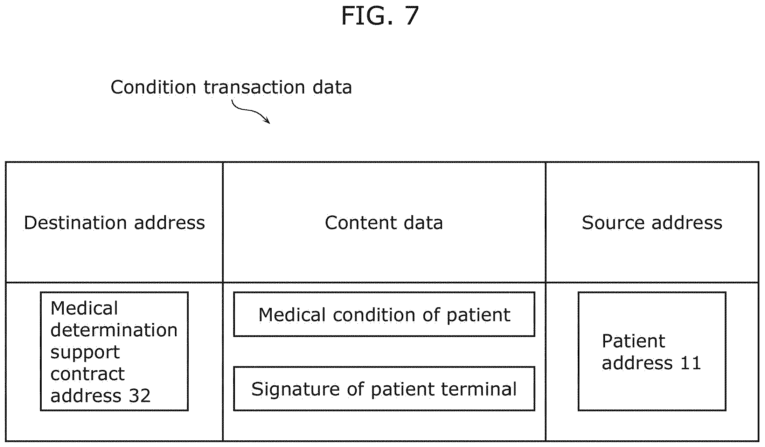

FIG. 7 conceptionally shows a data structure of condition transaction data according to this embodiment.

As shown in FIG. 7, the condition transaction data includes a destination address, content data, and a source address. The destination address includes medical determination support contract address 32. The source address includes patient address 11. The content data includes information indicating the medical condition of the patient and the signature of the source, patient terminal 10. The information indicating the medical condition of the patient corresponds to, for example, the medical facts indicating the medical condition of the patient including the vital data on the patient. The signature is used for verifying the authenticity of the condition transaction data. The signature may be, for example, obtained by encrypting a digest value of the value indicating the medical condition of the patient with a private key. In this case, a public key may be stored extra in the distributed ledger and open to the public.

Patient terminal 10 generates condition transaction data with such a data structure, and transmits the generated condition transaction data to medical determination support contract address 32.

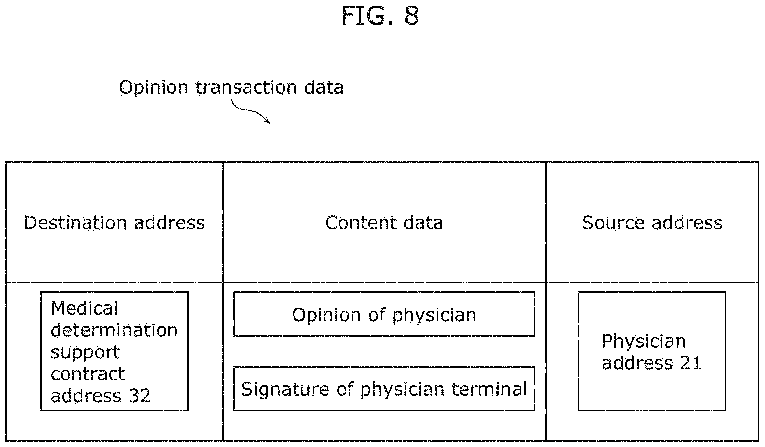

FIG. 8 conceptionally shows a data structure of opinion transaction data according to this embodiment.

As shown in FIG. 8, the opinion transaction data includes a destination address, content data, and a source address. The destination address includes medical determination support contract address 32. The source address includes physician address 21. The content data includes information indicating the opinion of the physician and the signature of the source, physician terminal 20. The information indicating the opinion of the physician is, for example, a result of a diagnosis by the physician based on the medical condition of the patient. The information further includes an opinion for executing the medical determination support smart contract based on the medical facts indicating the medical condition of the patient. The signature is used for verifying the authenticity of the opinion transaction data. The signature may be, for example, obtained by encrypting a digest value of the value indicating the opinion of the physician with a private key. In this case, a public key may be stored extra in the distributed ledger and open to the public.

Physician terminal 20 generates opinion transaction data with such a data structure, and transmits the generated opinion transaction data to medical determination support contract address 32.

Such the condition transaction data and the opinion transaction data are transmitted to medical determination support contract address 32 to execute the medical determination support smart contract. Accordingly, the medical determination support smart contract outputs medical suggestion transaction data including medical suggestion information on a medical treatment to be given to the patient.

Although not shown, the medical suggestion transaction data includes a destination address, content data, and a source address. The content data includes medical suggestion information. The destination address includes physician address 21. The source address includes medical determination support contract address 32 or the blockchain address of the node executing the medical determination support smart contract.

Now, an outline of an algorithm of the medical determination support smart contract according to this embodiment will be described.

### [1.6 Algorithm of Medical Determination Support Smart Contract]

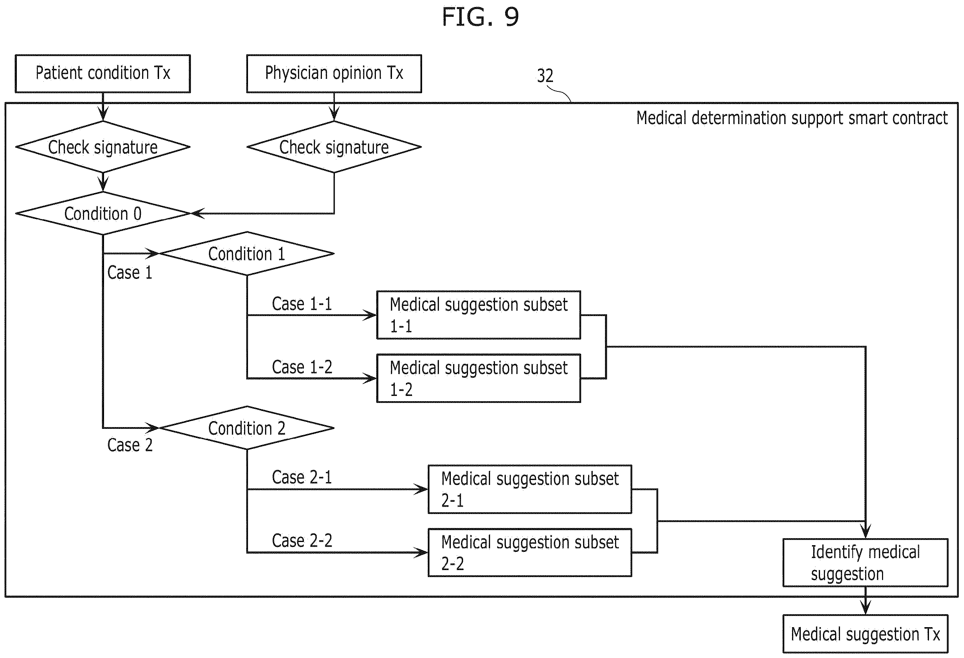

FIG. 9 conceptionally shows the algorithm of the medical determination support smart contract according to this embodiment.

As described above, the medical determination support smart contract is obtained by programming, in an executable manner, a determination on a medical treatment made as a result of a discussion between a patient and a physician. Being executed, the medical determination support smart contract outputs medical suggestion information on a medical treatment to be given to the patient. Here, executing the "determination on a medical treatment made as a result of a discussion between a patient and a physician" is as follows. The medical treatment to be given to the patient in view of the decision indicated in advance by the patient, in case where the patient himself/herself is unable to indicate his/her intention is determined and output. That is, the medical determination support smart contract is programmed as follows when being executed. A medical treatment to be given to the patient in view of the decision indicated in advance by the patient, in case where the patient himself/herself is unable to indicate his/her intention is determined. The determined medical treatment is then output as medical suggestion information.

More specifically, the algorithm of the medical determination support smart contract includes conditional branches and variables/constants as shown in FIG. 9. The conditional branches are indicated by conditions 0 to 2. The variables/constants indicate combinations of medical treatments represented by medical suggestion subsets 1-1 to 2-2 selected in accordance with the conditions. The algorithm of the medical determination support smart contract also includes a conditional expression using the following transaction data as inputs. One of the transaction data is the condition transaction data indicating the medical condition of the patient when the patient himself/herself is unable to indicate his/her intention. The other is the opinion transaction data indicating the opinion of the physician on the medical condition. The algorithm also includes conditional expressions so that the process proceeds to conditions 0 to 2 described above on the assumption that the signature included in the transaction data is validated. The algorithm of the medical determination support smart contract further includes a conditional expression that provides medical suggestion information on a medical treatment to be given to the patient as an output where these transaction data are input.

In other words, as shown in FIG. 9, the algorithm of the medical determination support smart contract includes the steps of: inputting the two transaction data described above, and checking the respective signatures included in the two transaction data. The algorithm of the medical determination support smart contract also includes the steps of: selecting medical suggestion subsets using three conditional branches indicated by conditions 0 to 2, and outputting the medical suggestion information.

Now, a processing flow of the algorithm of the medical determination support smart contract will be described.

First, transaction data on the medical condition of the patient and transaction data on an opinion of the physician are transmitted to medical determination support contract address 32. The inputting step of the medical determination support smart contract is then executed. In the inputting step, information indicating the medical condition of the patient included in the condition transaction data and information indicating the opinion of the physician included in the opinion transaction data are assigned to variables and held.

Next, the medical determination support smart contract executes the step of checking the signatures. The step of checking the signatures, whether the following signatures are identical is verified. The ones of the signatures are for verifying the authenticity of the transaction data, and registered as constants in the medical determination support smart contract. The others are included in the respective content data of the condition transaction data and the opinion transaction data. Note that the registered signatures for verifying the authenticity of the transaction data are registered as constants when deploying the medical determination support smart contract.

The next selecting step is executed only when the following is found as a result of the check in the step of checking the signatures. The registered signatures are identical with the signatures included in the respective content data of the condition transaction data and the opinion transaction data, that are, the input signatures. On the other hand, assume that the registered signatures and the input signatures are found to be unidentical as a result of the check. In this case, the processing is suspended and the variables of the medical determination support smart contract are initialized. That is, the execution of the medical determination support smart contract stops. Note that patient terminal 10 and physician terminal 20 may be notified of the stop of the execution of the medical determination support smart contract. In this case, the medical determination support smart contract may include patient address 11 and physician address 21 and generate alert transaction data indicating the stop of the execution.

In the step of selecting a medical suggestion subset, one of a plurality of medical suggestion subsets registered in advance is selected in accordance with conditional expressions registered in advance in the medical determination support smart contract. The conditional expressions registered in advance in the medical determination support smart contract are for executing a determination on a medical treatment made as a result of a discussion between the patient and the physician. The content data included in the condition transaction data and the opinion transaction data are assigned in the conditional expressions to select one of the medical suggestion subsets.

In example shown in FIG. 9, two cases are selected from the three conditional branches indicated by conditions 0 to 2. Thus, three conditional expressions and four medical suggestion subsets are registered in advance in the medical determination support smart contract.

In the step of outputting the medical suggestion information, medical suggestion transaction data is generated and output which includes, as the content data, the medical suggestion information indicating the medical suggestion subset selected in the selecting step. Here, the medical determination support smart contract generates the medical suggestion transaction data including physician address 21 as the destination address, and medical determination support contract address 32 as the source address.

The medical suggestion subset included as the content data in the one medical suggestion transaction data is shared with a physician via physician terminal 20. The physician gives a medical treatment according to this medical suggestion subset.

Note that the medical determination support smart contract may further include a step of recording a result of the medical treatment. Instead, a smart contract different from the medical determination support smart contract may describe the step of recording a result of the medical treatment. In the use of any smart contract, as in the step of checking the signatures described above, the signatures of the medical suggestion transaction data and the transaction data indicating the medical treatment given by the physician based on the medical suggestion subset may be checked first. The medical suggestion subset indicated by the medical suggestion transaction data may be, in association with the medical treatment given by the physician, recorded (i.e., stored) in the distributed ledge.

### [Example 1]

Now, a case will be described where a medical treatment subset according to the current POLST form is registered, as a medical treatment determined as a result of a discussion between a patient and a physician, in advance in the medical determination support smart contract.

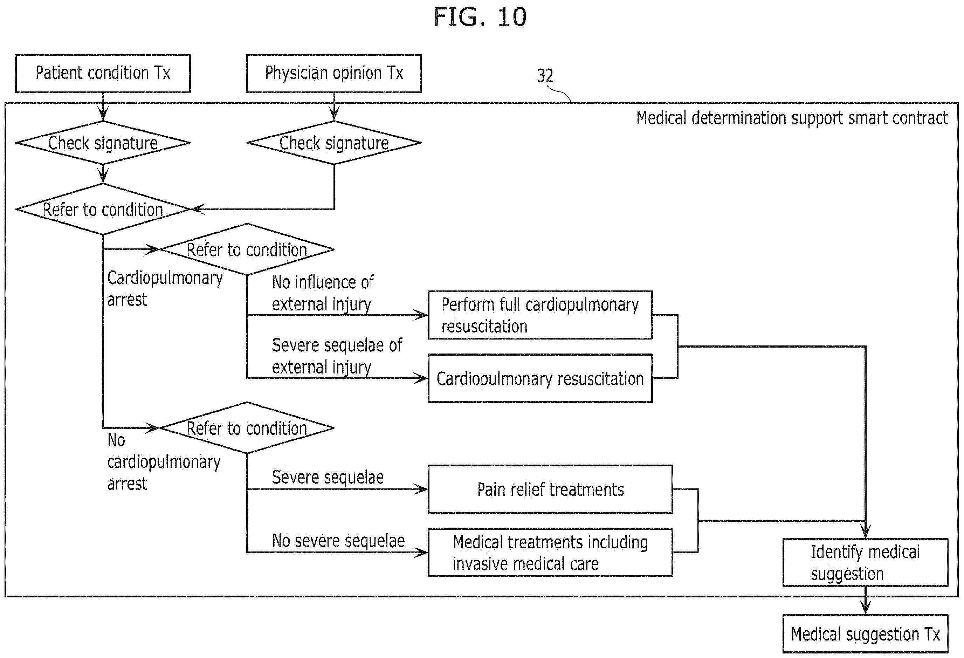

FIG. 10 conceptionally shows an algorithm of a medical determination support smart contract according to Example 1. FIG. 10 conceptionally shows an algorithm for selecting a medical treatment subset according to the current POLST form by executing a medical determination support smart contract.

First, transaction data on the medical condition of the patient and transaction data on an opinion of the physician are transmitted to medical determination support contract address 32. The inputting step of the medical determination support smart contract is then executed. In the inputting step, the following information are held as input values. One of the information indicates the medical condition of the patient when the patient himself/herself is unable to indicate his/her intention. The other information indicates the opinion of the physician on the patient under such circumstances.

Next, the medical determination support smart contract executes the step of checking the signatures. Here, whether the registered signatures are identical to the input signatures included in the respective content data of the condition transaction data and the opinion transaction data is checked to verify the reliability of the input values.

After that, the medical determination support smart contract executes the selecting step. If the indicated medical condition of the patient include a cardiopulmonary arrest, previous conditions are referred to. Depending on whether there may be an influence of an external injury, a medical suggestion subset is selected which is according to the medical treatment discussed in advance between the patient and the physician. For example, if the physician has the opinion that the patient has a cardiopulmonary arrest and may not be affected by an external injury, to "perform full cardiopulmonary resuscitation" is selected as the medical suggestion subset. For example, if the physician has the opinion that the patient has a cardiopulmonary arrest and may suffer from severe sequelae of an external injury, to perform soft "cardiopulmonary resuscitation" is selected as the medical suggestion subset.

On the other hand, if the indicated medical condition of the patient include no cardiopulmonary arrest, previous conditions are referred to. Depending on whether there may be severe sequelae, a medical suggestion subset is selected which is according to the medical treatment discussed in advance between the patient and the physician. For example, if the physician has the opinion that the patient has no cardiopulmonary arrest and may suffer from severe sequelae, to "pain relief treatments" is selected as the medical suggestion subset. For example, if the physician has the opinion that the patient has no cardiopulmonary arrest and may not suffer from severe sequelae, to "medical treatments including invasive medical care" is selected as the medical suggestion subset.

Note that each medical treatment subset registered in advance in the medical determination support smart contract holds a unique identifier. With the use of this identifier, the medical treatment indicated by the selected medical treatment subset is identified. Thus, the medical determination support smart contract transfers the identifier indicating the selected medical treatment subset to the outputting step.

In the outputting step, medical suggestion transaction data is generated and output which includes, as content data, medical suggestion information in the form of an identifier indicating the medical suggestion subset selected in the selecting step.

Accordingly, the physician gives a medical treatment based on the medical suggestion subset indicated by the medical suggestion transaction data to give a medical treatment in view of the decision indicated in advance by the patient.

Note that the medical determination support smart contract may further include a step of recording a result of the medical treatment (not shown). In this case, assume that transaction data is issued which includes physician address 21 as a source address, and the result of the medical treatment as content data. The medical determination support smart contract may record the identifier indicating the selected one of the medical suggestion subsets and the result of the medical treatment in association with each other in the distributed ledger in the recording step.

### [Example 2]

Now, a case will be described where a medical treatment subset according to a current living will form is, as a medical treatment determined as a result of a discussion between a patient and a physician, registered in advance in the medical determination support smart contract. Note that the description of the same points as in Example 1 will be omitted below.

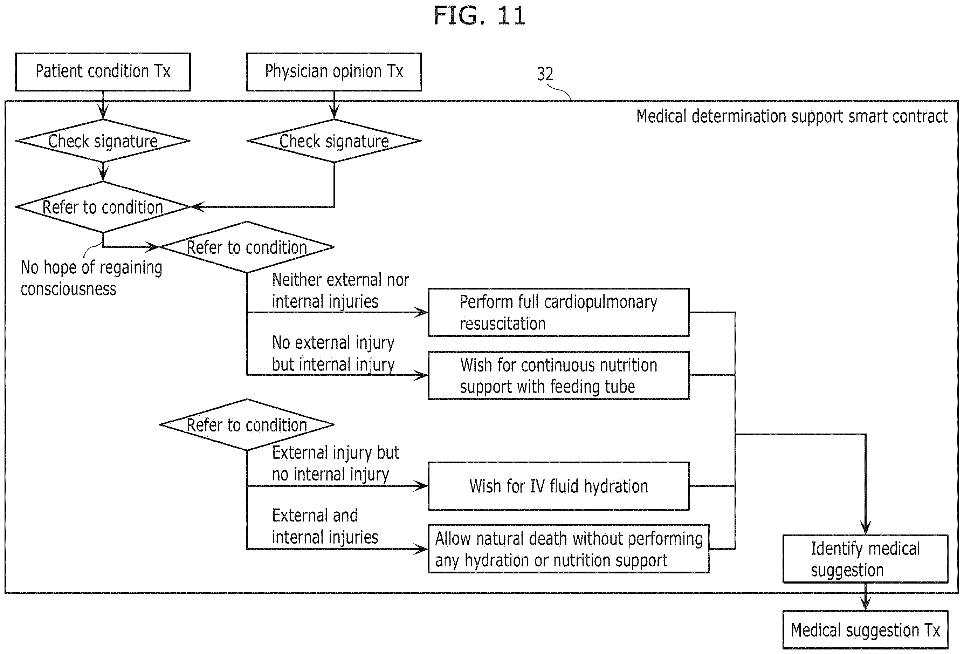

FIG. 11 conceptionally shows an algorithm of a medical determination support smart contract according to Example 2. FIG. 11 conceptionally shows an algorithm for selecting a medical treatment subset according to a current living will form by executing a medical determination support smart contract.

First, transaction data on the medical condition of the patient and transaction data on an opinion of the physician are transmitted to medical determination support contract address 32. The inputting step of the medical determination support smart contract is then executed. In the inputting step, the following information are held as input values. One of the information indicates the medical condition of the patient when the patient himself/herself is unable to indicate his/her intention. The other information indicates the opinion of the physician on the patient under such circumstances.

Next, the medical determination support smart contract executes the step of checking the signatures. Here, whether the registered signatures are identical to the input signatures included in the respective content data of the condition transaction data and the opinion transaction data is checked to verify the reliability of the input values.

After that, the medical determination support smart contract executes the selecting step. As the medical condition, the patient has no hope of regaining consciousness, previous conditions are referred to. Depending on whether there may be external and internal injuries, a medical suggestion subset is selected which is according to the medical treatment discussed in advance between the patient and the physician.

For example, if the physician has the opinion that the patient has no hope of regaining consciousness and has neither external nor internal injuries, to "perform full cardiopulmonary resuscitation" is selected as the medical suggestion subset. For example, if the physician has the opinion that the patient has no hope of regaining consciousness and has no external injury but an internal injury, to "wish for continuous nutrition support with feeding tube" is selected as the medical suggestion subset.

For example, if the physician has the opinion that the patient has no hope of regaining consciousness and has an external injury but no internal injury, to "wish for IV fluid hydration" is selected as the medical suggestion subset. For example, if the physician has the opinion that the patient has no hope of regaining consciousness, and has both an external injury and an internal injury, to "allow natural death without performing any hydration or nutrition support" is selected as the medical suggestion subset.

The processing flows of the algorithms of the medical determination support smart contract based on the current POLST form and the current living will form have been described above in Eexamples 1 and 2. The processing is however not limited thereto.

The medical determination support smart contract may further include a processing flow of selecting the details of the treatment in accordance with the costs of the medical treatment. The medical determination support smart contract may also include a processing flow of referring to the insurance or account balance of the patient himself/herself, when executing the processing flow of selecting the details of the treatment in accordance with the costs of the medical treatment.

### [1.7 Configuration of Server Device]

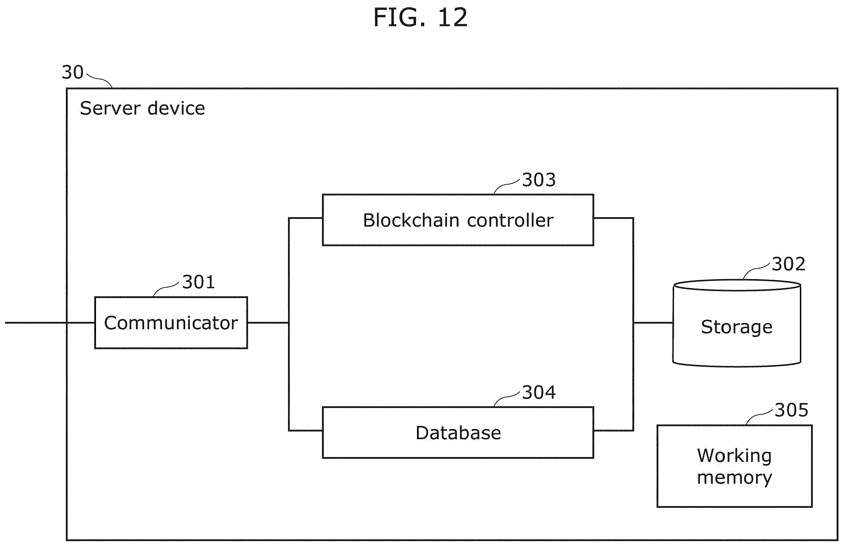

FIG. 12 is a block diagram showing an example configuration of server device 30 according to this embodiment.

As shown in FIG. 12, server device 30 includes communicator 301, storage 302, blockchain controller 303, database 304, and working memory 305. Server device 30 is operated by a processor executing a predetermined program using a memory. Now, the constituent elements will be described.

### <Communicator 301>

Communicator 301 communicates via communication network 40 with patient terminal 10 and physician terminal 20 with respect to the medical facts. Communicator 301 may record the medical facts received by server device 30 in storage 302.

In this embodiment, communicator 301 obtains, for example, third transaction data or condition transaction data including a medical determination support smart contract from patient terminal 10. Communicator 301 also obtains, from physician terminal 20, opinion transaction data and medical treatment result transaction data. In addition, communicator 301 transmits, to physician terminal 20, the medical suggestion transaction data output from the medical determination support smart contract.

### <Storage 302>

Storage 302 stores, for example, the medical suggestion transaction data output from the medical determination support smart contract. Storage 302 stores a result of control by blockchain controller 303.

Note that storage 302 may be a storage device shown in FIG. 1 including a distributed ledger. In this case, defined in storage 302 may be a distributed ledger area which includes the distributed ledger. Note that storage 302 may include working memory 305. In this case, defined in storage 302 may be a working memory area.

### <Blockchain Controller 303>

Blockchain controller 303 controls the establishment of the blockchain network, the generation of transaction data, the synchronization of the distributed ledger, and the execution of a consensus algorithm. Blockchain controller 303 also controls the storage of the respective control results in storage 302.

In this embodiment, blockchain controller 303 executes the medical determination support smart contract based on the following information. One of the information is biological information indicating the patient and included in the condition transaction data. The other indicates a result of a diagnosis by the physician based on the biological information and included in the opinion transaction data. Blockchain controller 303 also executes the medical determination support smart contract, and outputs suggestion information indicating a medical treatment to be given to the patient and obtained from the medical determination support smart contract.

Blockchain controller 303 also executes the consensus algorithm, and stores, in the distributed ledger, the medical treatment result transaction data obtained from physician terminal 20.

Blockchain controller 303 associates the medical treatment result transaction data obtained from physician terminal 20 with the medical suggestion transaction data output from the medical determination support smart contract. More specifically, blockchain controller 303 causes storage 302 to store the following information. The information associates a result of a medical treatment included in the medical treatment result transaction data with a medical suggestion subset included in the medical suggestion transaction data. Note that blockchain controller 303 may cause the distributed ledger to store the information associating the result of the medical treatment with the medical suggestion subset, by executing a smart contract different from the medical determination support smart contract.

Blockchain controller 303 perform other controls like blockchain controller 104 of patient terminal 10. The detailed description thereof will be omitted here.

### <Database 304>

Database 304 functions as a database of the medical facts. Database 304 may record medical information, such as prescription data, as the medical facts.

For example, database 304 transmits the following data as the medical suggestion subset via communicator 301 to patient terminal 10 and physician terminal 20. The data indicates a combination of medical treatments suggestable among the medical facts. Note that database 304 manages the medical facts indicated by the content data included in the transaction received by blockchain controller 303, and stores the managed medical facts in storage 302.

### <Working Memory 305>

Working memory 305 functions as an on-memory of server device 30. Working memory 305 reads a block recorded in the distributed ledger to hold the smart contract, and activates the smart contract. Note that working memory 305 may hold a contract address indicating the smart contract.

In this embodiment, working memory 305 may hold medical determination support contract address 32 that is the contract address indicating the medical determination support smart contract. In addition, working memory 305 may hold the medical determination support smart contract being activated.

### [1.8 Operation of Medical Treatment Management System]

Now, an outline of an operation of medical treatment management system 1 configured as described above will be described.

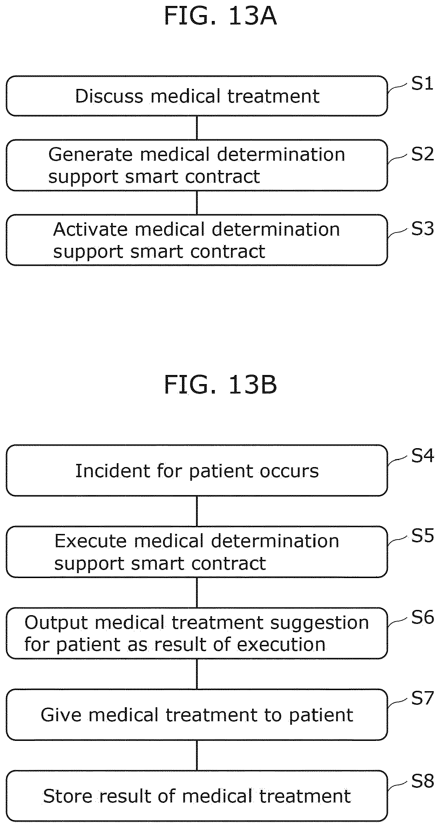

FIG. 13A shows an outline of an operation until storing the decision indicated by a patient in the distributed ledger of medical treatment management system 1 according to this embodiment.

First, in step S1, medical treatment management system 1 causes the patient and a physician to discuss a medical treatment. More specifically, medical treatment management system 1 causes the patient and the physician to discuss a medical treatment according to the intention of the patient for a case where the patient himself/herself is unable to indicate his/her intention.

In this embodiment, patient terminal 10 obtains a medical treatment subset according to the current POLST form, for example. Patient terminal 10 displays questions, for example, for making a decision on a medical treatment to be indicated in advance by the patient for the case where the patient is unable to indicate his/her intention. While discussing with the physician, the patient answers questions displayed by patient terminal 10 to make a decision on a medical treatment according to the intention of the patient for a case where the patient himself/herself is unable to indicate his/her intention. The determined medical treatment is the determination on a medical treatment made as a result of the discussion between the patient and the physician according to the decision indicated in advance by the patient himself/herself.

Next, in step S2, medical treatment management system 1 generates the medical determination support smart contract based on the result of the discussion on the medical treatment in step S2. More specifically, medical treatment management system 1 generates the medical determination support smart contract obtained by programming, in an executable manner, the determination on the medical treatment made as the result of the discussion between the patient and the physician.

Then, in step S3, medical treatment management system 1 activates the medical determination support smart contract generated in step S2. More specifically, medical treatment management system 1 stores, in the distributed ledger, the medical determination support smart contract generated in step S2. Medical treatment management system 1 then deploys the medical determination support smart contract to activate the medical determination support smart contract.

In this manner, medical treatment management system 1 stores the following medical determination support smart contract in the distributed ledger, and activate s the stored medical determination support smart contract. The medical determination support smart contract is obtained by programming, in an executable manner, the determination on the medical treatment made as the result of the discussion between the patient and the physician. Accordingly, the decision by the patient is stored in the distributed ledger.

FIG. 13B shows an outline of an operation until recording a result of a medical treatment in the following manner. Whether a medical treatment has been determined and given in accordance with the decision by the patient stored in the distributed ledger of medical treatment management system 1 according to this embodiment can be verified.

First, in step S4, assume that an incident occurs in which the patient requires a medical treatment. Here, the occurrence of the incident means as follows. The patient who has discussed a medical treatment with the physician in step S1 requires a medical treatment and the use of medical treatment management system 1 because the vital data on the patient indicates an abnormal value, for example. In this case, medical treatment management system 1 generates condition transaction data on the patient and opinion transaction data. The condition transaction data includes sensor information. The opinion transaction data includes information on a result of a diagnosis by the physician based on biological information on the patient.

Next, in step S5, the medical determination support smart contract is executed. More specifically, medical treatment management system 1 stores, in the distributed ledger, the condition transaction data and the opinion transaction data generated in step S4 to execute the medical determination support smart contract.

Then, in step S6, as a result of executing the medical determination support smart contract, a medical treatment suggestion for the patient is output. More specifically, the medical determination support smart contract is the suggestion information uniquely determined, using the condition transaction data and the opinion transaction data as inputs. The suggestion information on a medical treatment to be given to the patient is output.

After that, in step S7, based on the suggestion information on the medical treatment to be given to the patient as output in step S6, the physician gives a medical treatment to the patient. The physician inputs, to medical treatment management system 1, information on the medical treatment given to the patient as a result of the medical treatment.

At the end, in step S8, the result of the medical treatment in step S7 is stored. More specifically, medical treatment management system 1 stores, in the distributed ledger, the result of the medical treatment in step S7 in association with the suggestion information on the medical treatment to be given to the patient as output in step S6.

In this manner, medical treatment management system 1 ensures the resistance to the falsification and reliability of the medical treatment process reflecting the decision by the patient. Specifically, medical treatment management system 1 records, in a blockchain which is not easily falsified, a determination on a medical treatment including the decision indicated by the patient and the result of the medical treatment based on the result of the determination on the medical treatment, which can be referred to. Accordingly, whether a medical treatment has been determined and given in view of the decision indicated in advance by the patient can be verified.

### [1.8.1 Sequence of Deploying Medical Determination Support Smart Contract]

Now, a sequence until generating and deploying a medical determination support smart contract in medical treatment management system 1 will be described.

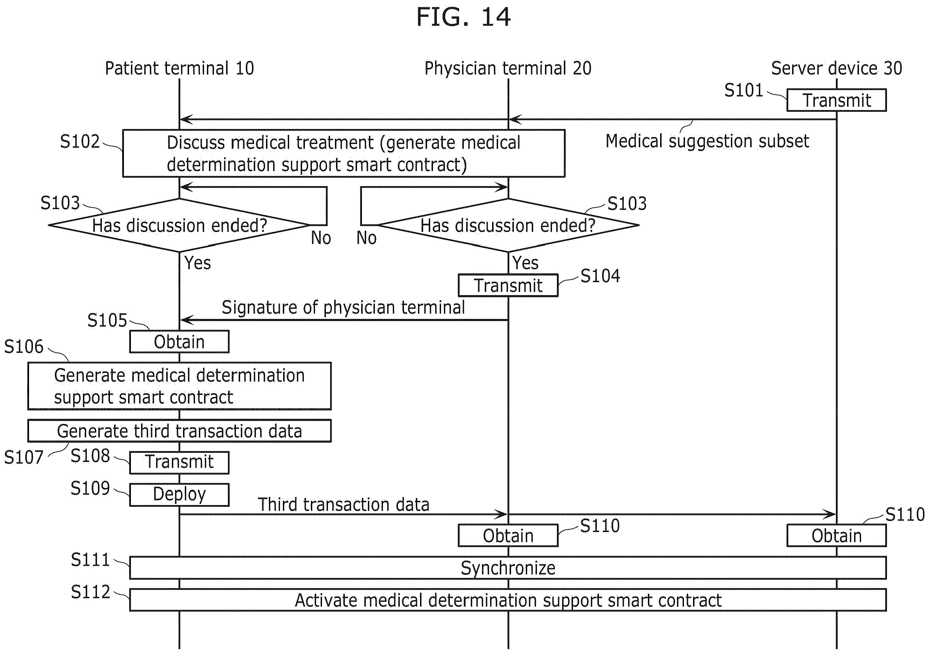

FIG. 14 shows the sequence of generating and activating the medical determination support smart contract by medical treatment management system 1 according to this embodiment.

First, server device 30 transmits medical suggestion subsets (S101). In this embodiment, server device 30 transmits medical suggestion subsets indicating medical facts according to a living will form or a POLST form used by a patient to indicate his/her intention in advance, and combinations of medical treatments.

Next, the patient using patient terminal 10 and a physician using physician terminal 20 discuss a medical treatment (S102). Here, the following determination is made by the discussion between the patient and the physician. The determination relates to a medical treatment to be used for generating a medical determination support smart contract as well. That is, the determination on a medical treatment is made in view of the decision indicated in advance by the patient himself/herself. In this embodiment, patient terminal 10 provides questions while showing medical facts according to the living will form or the POLST form and the combinations of the medical treatments to the patient. While discussing the physician, the patient answers the questions to make a determination on a medical treatment. Note that patient terminal 10 generates the medical determination support smart contract based on the made determination on the medical treatment, while making determination on the medical treatment. The details are as described in step S1 in FIG. 13A and the description is thus omitted here.

Then, patient terminal 10 and physician terminal 20 determine whether the discussion in step S102 has ended (S103).

If the discussion in step S102 is determined to have ended in step S103 (Yes in S103), physician terminal 20 transmits the signature of physician terminal 20 (S104).

Patient terminal 10 then obtains the signature of physician terminal 20 which has been transmitted in step S104 (S105).

Next, patient terminal 10 generates the medical determination support smart contract based on the determination on a medical treatment made as a result of the discussion in step S102 between the patient and the physician (S106). In this embodiment, during the discussion in step S102, patient terminal 10 causes the patient to make a determination on a medical treatment and generates (a draft of) the medical determination support smart contract. Accordingly, patient terminal 10 obtains the signature of physician terminal 20 and programs, for example, a binary program of the medical determination support smart contract to complete the generation of the medical determination support smart contract.

Then, patient terminal 10 generates third transaction data including the medical determination support smart contract generated in step S106 (S107). In this embodiment, patient terminal 10 generates, for example, the following third transaction data as shown in FIG. 6. The third transaction data includes the generated medical determination support smart contract in the form of the binarized program as the content data, and patient address 11 as a destination address and a source address.

After that, patient terminal 10 transmits the third transaction data generated in step S107 to patient terminal 10 itself (S108) to deploy the medical determination support smart contract on the blockchain network including patient terminal 10 (S109).

Next, physician terminal 20 and server device 30 obtain the third transaction data deployed and transmitted in step S109 (S110).

Then, patient terminal 10, physician terminal 20, and server device 30 are synchronized with each other. More specifically, each of patient terminal 10, physician terminal 20, and server device 30 executes the consensus algorithm, takes the third transaction data obtained in step S110 in a block, and stores the block in the distributed ledger.

After that, patient terminal 10, physician terminal 20, and server device 30 activate the medical determination support smart contract (S112).

### [1.8.2 Sequence of Executing Medical Determination Support Smart Contract]

Subsequently, a sequence until executing a medical determination support smart contract, outputting a medical suggestion, and recording the result of the medical treatment by physician, using medical treatment management system 1 will be described.

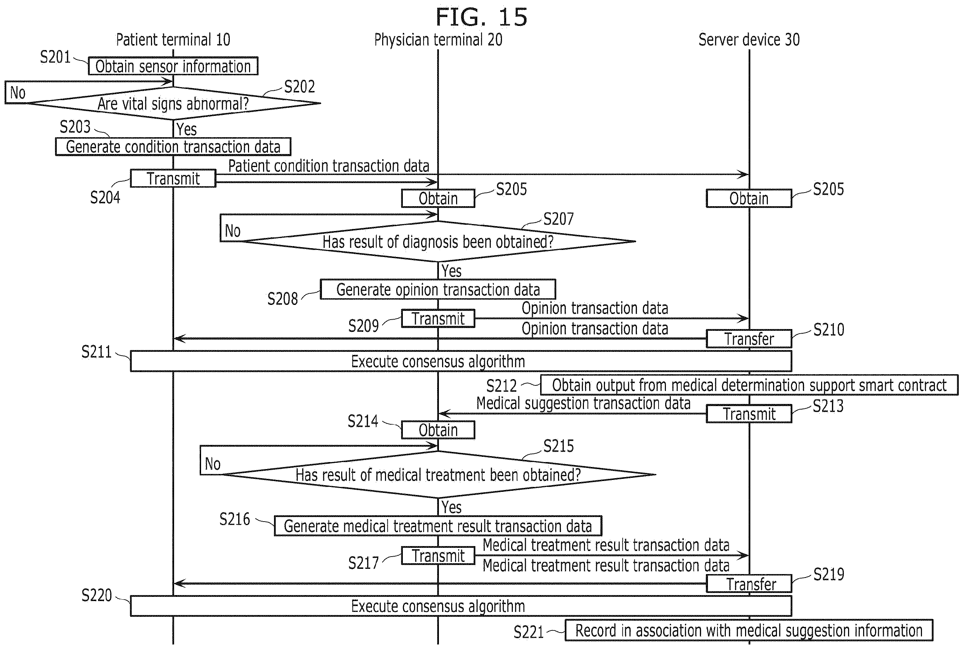

FIG. 15 shows a sequence until executing the medical determination support smart contract and recording the result of the medical treatment according to this embodiment.

First, patient terminal 10 obtains sensor information such as the biological information on the patient (S201). Specifically, patient terminal 10 obtains the sensor information, such as the biological information on the patient, from the patient or a monitoring device attached to the patient, for example.

Next, patient terminal 10 checks whether the vital signs of the patient are abnormal based on the sensor information obtained in step S201 (S202).

If the vital signs of the patient are determined to be abnormal in step S202 (Yes in S202), patient terminal 10 generates condition transaction data including the sensor information on the patient indicating the medical condition of the patient (S203). Note that "Yes in step S202", that is, the case where "the vital signs of the patient are abnormal" corresponds to the "occurrence of an incident" in step S4 of FIG. 13B described above. In this embodiment, patient terminal 10 generates the following condition transaction data, for example, as shown in FIG. 7. The condition transaction data includes the medical condition of the patient and the signature of patient terminal 10 as the content data, medical determination support contract address 32 as a destination address, and patient address 11 as a source address.

Patient terminal 10 then transmit the condition transaction data generated in step S203 to physician terminal 20 and server device 30 by broadcasting (S204).

After that, physician terminal 20 and server device 30 obtain the condition transaction data (S205).

Next, physician terminal 20 checks whether a result of a diagnosis by the physician based on the medical condition of the patient included in the condition transaction data has been obtained (S207).

If a result of a diagnosis is determined to have been obtained in step S207 (Yes in S207), physician terminal 20 generates the following opinion transaction data (S209). The opinion transaction data indicates the opinion of the physician and includes information on the result of the diagnosis by the physician based on the biological information on the patient. In this embodiment, physician terminal 20 generates the following opinion transaction data, for example, as shown in FIG. 8. The opinion transaction data includes the opinion of the physician and the signature of physician terminal 20 as content data, medical determination support contract address 32 as a destination address, and physician address 21 as a source address.

Physician terminal 20 then transmits the opinion transaction data generated in step S208 to server device 30 (S209).

After that, server device 30 transfers the condition transaction data transmitted in step S209 to patient terminal 10 (S210). Note that physician terminal 20 may transmit the opinion transaction data generated in step S208 to patient terminal 10 and server device 30 by broadcasting. In this case, step S210 is not performed.

Next, patient terminal 10, physician terminal 20, and server device 30 execute the consensus algorithm (S211). More specifically, after verifying (i.e., validating) the condition transaction data and the opinion transaction data, each of patient terminal 10, physician terminal 20, and server device 30 generates a block including the condition transaction data and the opinion transaction data. Each of patient terminal 10, physician terminal 20, and server device 30 then stores the block including the condition transaction data and the opinion transaction data in the distributed ledger. Each of patient terminal 10, physician terminal 20, and server device 30 inputs, to the medical determination support smart contract, the condition transaction data and the opinion transaction data taken into the block. Here, the medical determination support smart contract is stored in the distributed ledger, and written in the working memory to be activated. Accordingly, the medical determination support smart contract is executed using the condition transaction data and the opinion transaction data as input values.

Server device 30 then obtains an output from the medical determination support smart contract (S212).

After that, server device 30 transmits the medical suggestion transaction data obtained in step S212 to physician terminal 20 (S213).

Next, physician terminal 20 obtains the medical suggestion transaction data transmitted in step S213 (S214). The physician using physician terminal 20 gives a medical treatment to the patient based on suggestion information included in the obtained medical suggestion transaction data. The physician then inputs, to physician terminal 20, the information on the medical treatment given to the patient as the result of the medical treatment. Note that server device 30 and physician terminal 20 may record the obtained medical suggestion transaction data extra in the distributed ledger, together with patient terminal 10.

After that, physician terminal 20 checks whether the result of the medical treatment has been obtained (S215).

If the result of the medical treatment is determined to have been obtained in step S215 (Yes in S215), physician terminal 20 generates medical treatment result transaction data including the result of the medical treatment (S216).

Next, physician terminal 20 transmits the medical treatment result transaction data generated in step S216 to server device 30 (S218).

Server device 30 then transfers, to patient terminal 10, the medical treatment result transaction data transmitted in step S218 (S219). Note that physician terminal 20 may transmit the medical treatment result transaction data generated in step S216 to patient terminal 10 and server device 30 by broadcasting. In this case, step S219 is not performed.

After that, patient terminal 10, physician terminal 20, and server device 30 execute the consensus algorithm (S220). More specifically, after verifying (i.e., validating) of the medical treatment result transaction data, each of patient terminal 10, physician terminal 20, and server device 30 generates a block including the medical treatment result transaction data. Each of patient terminal 10, physician terminal 20, and server device 30 then stores the block including the medical treatment result transaction data in the distributed ledger.

Next, server device 30 records the medical suggestion information and the result of the medical treatment in association with each other, out of the medical treatment result transaction data and the medical suggestion transaction data stored in the distributed ledger (S221). Assume that a smart contract for recording the medical suggestion information and the result of the medical treatment in association with each other is stored in the distributed ledger, and written in a working memory to be activated. In this case, server device 30 may input, to the smart contract, the medical treatment result transaction data taken into the block. Accordingly, the mart contract is executed using the medical treatment result transaction data and the medical suggestion transaction data as input values. The medical suggestion information and the result of the medical treatment are recorded in association with each other.

### [1.10 Advantages]

As described above, the control method, for example, according to the present disclosure records, in the distributed ledger, every information including medical facts for determining a medical treatment in case where a patient himself/herself is unable to indicate his/her intention. The control method, for example, according to the present disclosure causes a medical determination support smart contract to suggest a medical treatment in case where the patient himself/herself is unable to indicate his/her intention. The medical determination support smart contract uniquely determines a medical treatment based on the medical condition or other factors of the patient in view of the decision indicated in advance by the patient. It is thus difficult to falsify any information including the medical determination support smart contract, that is, any information for determining a medical treatment in case where the patient himself/herself is unable to indicate his/her intention. Accordingly, whether a medical treatment has been determined and given in view of the decision indicated in advance by the patient can be verified. In addition, the distributed ledger makes the falsification of any information difficult, which reduces attacks through unauthorized accesses to the medical information. Accordingly, whether a medical treatment has been determined and given in view of the decision indicated in advance by the patient can be verified highly reliably.

### [Variations]

Note that the present disclosure has been described above based on the embodiment. Needless to mention, the present disclosure is however not limited to the embodiment described above. The present disclosure includes the following cases.
(1) The embodiment has been described above where patient terminal 10 includes a single blockchain controller. The configuration is however not limited thereto. Patient terminal 10 may include a plurality of blockchain controllers. Alternatively, for example, a monitor device connected to patient terminal 10 may include a blockchain controller. Alternatively, these may be combined.
(2) An example has been described above in the embodiment where single patient terminal 10, single physician terminal 20, and single server device 30 constitute the blockchain network. The configuration is however not limited thereto. For example, a plurality of patient terminals 10, a plurality of physician terminals 20, and a plurality of server devices may constitute the blockchain network. Alternatively, one element and a plurality of elements such as single patient terminal 10, a plurality of physician terminals 20, and a plurality of server devices may be combined to constitute the blockchain network.
(3) An example has been described above in the embodiment where server device 30 includes database 304. The configuration is however not limited thereto. For example, a plurality of server devices 30 may have divisions of single database 304.
(4) The embodiment has been described above where patient terminal 10 generates a medical determination support smart contract. The configuration is however not limited thereto. Patient terminal 10 may generate a medical determination support smart contract in cooperation with physician terminal 20. Alternatively, physician terminal 20 may generate a medical determination support smart contract in cooperation with patient terminal 10. In this case, the signatures of patient terminal 10 and physician terminal 20 may be required as a multi-signature to issue transaction data including the medical determination support smart contract.
(5) An example has been described above in the embodiment where Ethereum is used as a platform of the blockchain forming the distributed ledger, that is, as a blockchain platform. The configuration is however not limited thereto. Any platform including Hyperledger Fabric, for example, may be used as long as capable of describing a smart contract on a blockchain. The type of a platform is not limited.
(6) An example has been described above in the embodiment where Solidity, which is the language for programming Ethereum, is used as the language for programming a smart contract. The configuration is however not limited thereto. Any programming language may be used as long as capable of describing a smart contract on a selected blockchain platform.
(7) An example has been described above in the embodiment where a smart contract included in transaction data is in the form of binary data. The configuration is however not limited thereto. Any data format including an application binary interface, for example, may be used as long as capable of executing a selected blockchain platform.
(8) In the embodiment described above, the medical facts are included in transaction data to be received and transmitted. The configuration is however not limited thereto. The medical facts may be received and transmitted in any form allowing communications among patient terminal 10, physician terminal 20, and server device 30. For example, the medical facts may be received and transmitted using P2P file transportation or through communications on a website.
(9) The embodiment has been described above where the inputs to the medical determination support smart contract are the condition transaction data transmitted by patient terminal 10 and the opinion transaction data transmitted by physician terminal 20. The configuration is however not limited thereto. For example, physician terminal 20 may obtain the condition transaction data generated by patient terminal 10 and put the medical condition of the patient included in the condition transaction data into opinion transaction data to be used as an input. While an example has been described where the inputs to the medical determination support smart contract are the condition transaction data and the opinion transaction data, no division of the transaction data is designated. As long as corresponding to the algorithm in the medical determination support smart contract, single transaction data such as condition transaction data may be divided into two or more so as to be input.
(10) The embodiment has been described above where the inputs to the medical determination support smart contract are the condition transaction data transmitted by patient terminal 10 and the opinion transaction transmitted by physician terminal 20. The configuration is however not limited thereto. A smart contract including a medical determination support smart contract may refer to the data recorded in a distributed ledger including other smart contracts. The other smart contracts may include, for example, a smart contract indicating last will information on the patient himself/herself and a smart contract indicating information on the family of the patient. The other smart contracts may also include a smart contract indicating a determination by a third party described like a medical determination support smart contract. The other smart contracts may also include a smart contract indicating not only medical care during the life time but also an after-death treatment such as an organ donation described like a medical determination support smart contract. The other smart contracts may further include a smart contract obtained by combining the smart contracts described above. In these cases, the smart contract may describe, in advance, a smart contract to be referred to or transaction conditions.
(11) The embodiment has been described above where the processing is suspended, if any signature included in the transaction data is determined to be invalid in the processing of checking the signature(s) in the medical determination support smart contract. The processing is however not limited thereto. In this case, the medical determination support smart contract may generate transaction data indicating that the transaction data includes an invalid signature, and transmit the generated transaction data to patient terminal 10, physician terminal 20, and/or server device 30. The medical determination support smart contract may also perform the suspension processing described above together with the processing of generating and transmitting the transaction data indicating that the transaction data includes an invalid signature.
(12) Each of the devices in the embodiment described above may be a computer system including, specifically, a microprocessor, a ROM, a RAM, a hard disk unit, a display unit, a keyboard, or a mouse. The RAM or the hard disk unit stores computer programs. The microprocessor operates in accordance with the computer programs so that the devices fulfill their functions. The computer programs are here obtained by combining instruction codes indicating instructions to the computer to fulfill predetermined functions.
(13) Some or all of the constituent elements of each of the devices in the embodiment described above may serve as a single system large-scale integrated (LSI) circuit. The system LSI circuit is a super multifunctional LSI circuit manufactured by integrating a plurality of components on a single chip, and specifically is a computer system including a microprocessor, a ROM, and a RAM, for example. The RAM stores computer programs. The microprocessor operates in accordance with the computer programs so that the system LSI circuit fulfills its function.

The constituent elements of the devices may be configured as respective chips, or some or all of the constituent elements may be included into a single chip.

While the system LSI circuits are named here, the integrated circuits may be referred to ICs, LSI circuits, super LSI circuits, or ultra LSI circuits depending on the degree of integration. The circuit integration is not limited to the LSI. The devices may be dedicated circuits or general-purpose processors. A field programmable gate array (FPGA) programmable after the manufacture of an LSI circuit or a reconfigurable processor capable of reconfiguring the connections and settings of circuit cells inside an LSI may be employed.

Appearing as an alternative circuit integration technology to the LSI, another technology that progresses or deprives from the semiconductor technology may be used for integration of functional blocks. Biotechnology is also applicable.

(14) Some or all of the constituent elements of each of the devices described above may serve as an IC card or a single module detachably attached to the device. The IC card or the module is a computer system including a microprocessor, a ROM, and a RAM, for example. The IC card or the module may include the super multifunctional LSI circuit described above. The microprocessor operates in accordance with computer programs so that the IC card or the module fulfills its function. This IC card or this module may have a tamper resistance. The present disclosure may be directed to the method described above. The present disclosure may also be directed to computer programs causing a computer to execute this method or digital signals indicating the computer programs.

The present disclosure may be directed to a computer readable storage medium capable of recording computer programs or digital signals, for example, a flexible disk, a hard disk, a CD-ROM, an MO, a DVD, a DVD-ROM, a DVD-RAM, a Blu-ray Disk (BD, registered trademark), or a semiconductor memory, for example. The present disclosure may be directed to the digital signals stored in these recording media.

In the present disclosure, the computer programs or the digital signals may be transferred via telecommunication lines, wireless or wired communication lines, networks represented by the Internet, or data broadcasts, for example.

The present disclosure may be directed to a computer system including a microprocessor and a memory. The memory may store the computer programs described above, whereas the microprocessor may operate in accordance with the computer programs.

The programs or the digital signals may be stored in a storage medium and then transferred, or may be transferred via a network, so as to be executed by another independent computer system. The embodiment and variation described above may be combined.

### [Industrial Applicability]

The present disclosure is applicable to a control method, a program, and a control device, for example, to a control method, a program, and a control device for the following system. The system allows follow-up verification by safely managing and sharing medical facts including a patient's decision making and a result of diagnosing a patient, and managing every medical treatment data.

### [Reference Signs List]

- 1: medical treatment management system
- 10: patient terminal
- 11: patient address
- 20: physician terminal
- 21: physician address
- 30: server device
- 31: server address
- 32: medical determination support contract address
- 40: communication network
- 101, 201, 301: communicator
- 102: sensor information obtainer
- 103: smart contract generator
- 104, 203: blockchain controller
- 105, 204: display
- 106, 205, 302: storage
- 202: physician opinion generator
- 303: blockchain controller
- 304: database
- 305: working memory

## Claims

1. A control method to be executed by a system including a distributed ledger storing a smart contract obtained by programming, in an executable manner, a determination on a medical treatment made as a result of a discussion between a patient and a physician, the control method comprising:
obtaining first transaction data including biological information on the patient obtained by a sensor;
obtaining second transaction data including information on a result of a diagnosis by the physician based on the biological information;
executing the smart contract based on the biological information and the result of the diagnosis included in the first transaction data and the second transaction data obtained, respectively; and
outputting medical suggestion information indicating a medical treatment to be given to the patient, and obtained by executing the smart contract.

2. The control method according to claim 1, further comprising, before obtaining the first transaction data and the second transaction data:
obtaining third transaction data including the smart contract generated based on the determination on the medical treatment made as the result of the discussion between the patient and the physician; and
storing, in the distributed ledger, a block including the third transaction data.

3. The control method according to claim 2, wherein
the third transaction data includes a signature for verifying an authenticity of the third transaction data.

4. The control method according to any one of claims 1 to 3, wherein
the smart contract is a program including a description including a conditional expression for executing the determination on the medical treatment, using the first transaction data and the second transaction data as input values.

5. The control method according to any one of claims 1 to 4, wherein
each of the first transaction data and the second transaction data includes a contract address indicating an address of the smart contract.

6. The control method according to any one of claims 1 to 5, further comprising:
obtaining fourth transaction data including medical treatment result information which is information on a medical treatment given to the patient by the physician; and
storing, in the distributed ledger, the medical treatment result information in association with the medical suggestion information.

7. The control method according to any one of claims 1 to 6, wherein
the biological information is information indicating a cardiopulmonary arrest,
the result of the diagnosis indicates that there is no external injury, and
the medical suggestion information is information indicating that the medical treatment to be given to the patient is cardiopulmonary resuscitation.

8. The control method according to any one of claims 1 to 7, wherein
the distributed ledger is constructed on a blockchain platform.

9. A program for causing a computer to execute:
obtaining first transaction data including biological information on a patient obtained by a sensor;
obtaining second transaction data including information on a result of a diagnosis by a physician based on the biological information;
executing a smart contract based on the biological information and the result of the diagnosis included in the first transaction data and the second transaction data obtained, respectively, the smart contract being stored in a distributed ledger and obtained by programming, in an executable manner, a determination on a medical treatment made as a result of a discussion between the patient and the physician; and
outputting medical suggestion information indicating a medical treatment to be given to the patient, and obtained by executing the smart contract.

10. A control device comprising:
a processor;
a memory; and
a distributed ledger, wherein
the distributed ledger stores a smart contract obtained by programming, in an executable manner, a determination on a medical treatment made as a result of a discussion between a patient and a physician,
the processor obtains first transaction data including biological information on the patient obtained by a sensor,
the processor obtains second transaction data including information on a result of a diagnosis by the physician based on the biological information,
the processor executes the smart contract based on the biological information and the result of the diagnosis included in the first transaction data and the second transaction data obtained, respectively, using the memory, and
the processor outputs medical suggestion information indicating a medical treatment to be given to the patient, and obtained by executing the smart contract.
